# EUROPEAN PATENT APPLICATION

(11) **EP 4 005 672 A1**
(43) Date of publication of application: **01.06.2022**
(21) Application number: 20209897.6
(22) Date of filing: 25.11.2020
(51) Int. Cl.: B01L 3/00, C12M 3/00, G01N 33/487, C12N 5/071

(54) **A DEVICE FOR SEPARATING AND ANALYZING SEMINAL SAMPLE, SYSTEM AND METHOD**

(71) Applicant: Pera Labs Medikal Arastirma ve Gelistirme Limited Sirketi, 34467 Istanbul (TR)
(72) Inventor: Ozkosem, Burak, 34467 Istanbul (TR)
(74) Representative: Stellbrink & Partner Patentanwälte mbB

(57) **Abstract**

The invention relates to a device for analyzing a seminal sample, the device comprising a microvolume unit configured to allocate at least one fluid, wherein the microvolume unit comprises: at least one inlet to receive the at least one fluid, at least one outlet configured to expel the at least one fluid, and at least one channel arranged on the microvolume unit, wherein the at least one channel is configured to guide the at least one fluid through the microvolume unit. The invention also discloses a method for analyzing a seminal sample using a device, the method comprising introducing the seminal sample into the device, guiding the seminal sample through the device, segregating sperm cells from the seminal sample into a fluid medium, and recovering sperm cells from the fluidic medium. Moreover, the invention also relates to a system for analyzing seminal image data, the system comprising: a detecting component configured to detect sperm cells in the seminal sample, an analyzing component configured to analyze sperm cells in the seminal sample, a sorting component configured to sort sperm cells in the seminal sample, and at least one deep learning component. The invention also discloses an analytical method for evaluating male fertility using a system according to any of the preceding system claims, the method comprising: analyzing seminal sample, training data comprising at least one of seminal sample data, finding data, wherein the finding data comprises at least one known fertility statuses, detecting at least one feature within the training seminal sample data by means of the at least one deep learning component, and using a test data comprising seminal sample data from a test, wherein the analytical method comprises detecting the at least one feature by means of the at least one deep learning component, wherein the at least one feature is detected in test seminal sample data.

## Description

### Field

The invention lies in the field of biological sample analysis and particularly in the field of mammalian sample analysis. The goal of the invention is to provide a device for processing and analyzing seminal samples of mammals. More particularly, the present invention relates to a device for analyzing seminal samples, system for analyzing the seminal samples, a method performed in such a device and such a system and corresponding use of the device and the system.

### Introduction

Infertility is an increasing medical and social problem from which 8%-12% of couples suffer worldwide. Male factor infertility is an important cause of infertility, and approximately 40%-50% of all infertility cases are due to male infertility. Furthermore, the fertility rate among men younger than 30 years has decreased by 15% worldwide. A proper diagnosis of male factor infertility in an infertility analysis is dependent on an accurate measurement of semen quality. Semen quality analysis is the most frequently used diagnostic option and fundamental investigation in an infertility analysis. In addition to evaluating male infertility, semen analysis may also be used for post-vasectomy screening, and anyone who plans to preserve or donate sperm is expected to undergo such an examination. However, the shortcomings of conventional manual semen analysis (MSA), including that it is laboratory-based, labor intensive and subjective in nature, producing inconsistent reports that can cause misdiagnosis or delayed infertility treatment, have promoted the development of semi-computerized and computerized semen measuring devices, known as computer-assisted sperm analysis (CASA). The conventional CASA systems are semi-automated sperm tracking computer-aided devices, essentially focusing through the microscope to provide successive images of spermatozoa within static field of view. These systems use special software to extract desired information and produce the desired output. If appropriate protocols are followed, high precision and provision of quantitative data on sperm kinetics are two major advantages of CASA over MSA. In addition, CASA systems reduce the burden of measuring sperm tracks when individual tracking data are needed. Another advantage is that any test samples can be analyzed in a short time. Most CASA systems allow partial automation in routine sperm analysis, but they have also shown limited success due to poor accuracy at low and high sperm counts and the high cost of virtually all CASA devices. Moreover, different CASA systems use different mathematical algorithms to calculate various sperm quality parameters, leading to the greatest disadvantage in the clinical application of CASA, with unreliability of comparative parameters across all device. Although CASA provides quantitative data on sperm kinetics, most CASA systems still rely greatly on highly trained technicians and require an additional bulky component for data collection and computational units for data analysis. The factors influencing the accuracy of the results and the lack of quality control may lead to large variations between different CASA systems and laboratories

WO 2016 182 551 A1 discloses a method and system for anatomical landmark detection in medical images using deep neural networks is disclosed. For each of a plurality of image patches centered at a respective one of a plurality of voxels in the medical image, a subset of voxels within the image patch is input to a trained deep neural network based on a predetermined sampling pattern. A location of a target landmark in the medical image is detected using the trained deep neural network based on the subset of voxels input to the trained deep neural network from each of the plurality of image patches.

US 2016 0174902 A1 discloses a method and system for anatomical object detection using marginal space deep neural networks is disclosed. The pose parameter space for an anatomical object is divided into a series of marginal search spaces with increasing dimensionality. A respective sparse deep neural network is trained for each of the marginal search spaces, resulting in a series of trained sparse deep neural networks. Each of the trained sparse deep neural networks is trained by injecting sparsity into a deep neural network by removing filter weights of the deep neural network.

US 8 639 043 B2 discloses methods for classifying different parts of a sample into respective classes based on an image stack that may comprise one or more images.

US2017 0053 398 A1 discloses various arrangements for identifying and grading cancer in tissue samples. A digital image of a stained tissue sample may be acquired. A Shearlet transform may be performed on the digital image of the stained tissue sample. Shearlet coefficients may be calculated based on the performed Shearlet transform of the normalized digital RGB image of the stained tissue sample. A trained neural network may be applied to create a plurality of feature maps using the digital image and Shearlet coefficients, wherein the trained neural network was trained using a plurality of images and Shearlet coefficients of a plurality of digital images. A classifier may be applied to an output of the trained neural network to identify whether cancer is present in the stained tissue sample. A notification may be output that is indicative of a grade of detected cancer in the sample.

### Summary

In light of the above, it is therefore an object of the present invention to overcome or at least to alleviated the shortcomings and disadvantages of the prior art. More particularly, it is an object of the present invention to provide a device and a corresponding method for processing and analyzing seminal samples, wherein the device and the method are less prompt to error and capable of a more efficient batch analysis.

These objects are met by the present invention.

In a first aspect, the invention relates to a device for analyzing a seminal sample, the device comprising a microvolume unit configured to allocate at least one fluid, wherein the microvolume unit comprises at least one inlet to receive the at least one fluid, at least one outlet configured to expel the at least one fluid, and at least one channel arranged on the microvolume unit, wherein the at least one channel may be configured to guide the at least one fluid through the microvolume unit.

The device may comprise an inlet reservoir configured to hold the seminal sample. The seminal sample may comprise a raw-sperm sample.

The device may comprise an outlet reservoir for collecting the sperm sample, wherein the sperm sample may be separated from the raw sperm sample.

The at least one channel may comprise at least one selection channel arranged between the inlet reservoir and the outlet reservoir.

The inlet reservoir may be directly fluidic connected to at least one of: the least one inlet, and the outlet reservoir may be directly fluidic connect to at least one of: the least one outlet.

The at least one selection channel may be configured to provide a fluid connection between the inlet reservoir and the outlet reservoir.

The device may comprise a detection zone configured to allow the analysis of the separated seminal sample by means of the optical analytical device.

The device may comprise at least one separation wall may be arranged between the detection zone and the analytical device.

The Shear let at least one separation wall may comprise a transparent material configured to allow the at least light source passing through a portion of the separated sperm sample in the detection zone into the optical device.

The detection zone may be configured to allocate a known volume of the seminal sample to count the number of sperm cells in the portion of the detection zone.

The detection zone may comprise at least one reference comprising one of: an object of known dimensions, and a position.

The at least one reference may be configured to allow at least one of: scaling at one of: the at least one captured image data, and registering at least one of: the at least one captured image data.

The at least one selection channel may comprise a geometry configured to impede helical locomotion of a subpopulation of sperm, wherein the geometry may be configured to allow 2D slither swimming of a slither capable subset of sperm within the at least one selection channel.

The device may be further configured to simulate natural conditions of a female body via at least one of: a high-viscosity, and physiological buffer in a micro-network.

The at least one selection channel may comprise a height sufficiently low to restrict the helical locomotion of sperm within the at least one selection channel.

The at least one selection channel may comprise a channel height lower than 10µm, preferably lower than 5µm, more preferably lower than 3µm, such as 2µm.

The at least one selection channel may comprise a channel width between 10µm and 100um, preferably between 25µm and 150µm, more preferably between 50µm and 100µm.µm
The at least one selection channel a channel length between 1 and 10 cm, preferably between 3 and 8 cm, such as between 5 and 7 cm.

The at least one selection channel may be configured to allocate a volume of at least 0.01 mL, preferably at least 0.02 mL, more preferably at least 0.05 mL and less than 1 mL, preferably less than 0.8 mL, more preferably less than 0.5 mL.

The at least one selection channel may be configured to allocate a volume of raw sperm sample comprising a concentration of least 10 million sperm per mL, preferably at least 50 million sperm per mL, more preferably at least 100 million sperm per mL, and less than least 250 million sperm per mL, preferably less than 100 million sperm per mL, more preferably less than 150 million sperm per mL.

The at least one selection channel may comprise a single selection channel, wherein the single selection channel may comprise a ratio between the channel width and channel height of at least 2, preferably at least 4, more preferably at least 5.

The at least one selection channel may comprise at least one support structures arranged longitudinal along the channel length.

The at least one support structure may be configured to support and/or prevent a collapsing of the at least one single selection channel.

The device may comprise a hard material comprising at least one of: silicon, and an elastomeric material such as silicone rubber.

The device may comprise a semi conductive material comprising at least one of: polysilicon, silicon nitride, and silicon dioxide.

The device may comprise a metal and/or a metallic alloy comprising at least one of: magnetic, and electrically conductive.

The device may comprise at least one of: polydimethylsiloxane (PDMS), poly(methyl methacrylate) (PMMA), a suitable thermoplastic such as polycarbonates, polystyrene, and glass.

The device may comprise at least one valve configured to assume at least two operational states. The at least one valve may be configured to regulate the fluidic connection between the device and the macrofluidic chamber. The at least one valve may be configured to assume a first operational state and a second operational state, wherein the first operational state and different from the second operational state, wherein the at least one valve may be configured to: establish the fluid connection between the device and the microfluidic chamber while in the first operational state, discontinue the fluid connection between the device and the microfluid chamber while in the second operational state.

The at least one valve component may comprise at least one internal valve configured to regulate a flow within the device.

In one embodiment, at least one of: the at least one valve may be a check valve configured to allow flow of fluids in only one direction through a conduit comprised by the check valve

The at least one valve may be configured to individually and/or independently be selected to assume at least one of: the at least two operational state.

The device may comprise an actuation element. In one embodiment, actuation element may comprise an active actuation means comprising at least one of: air pressure, electrostatic forces, and magnetic forces. In another embodiment, the actuation element may comprise a passive actuation means comprising means to facilitate flow of the seminal sample, wherein the passive actuation means may comprise a viscosity-altering agent comprising at least one of: a liquefying agent and a surfactant.

The device may comprise at least one membrane.

The active actuation means may be applied from at least one of: a pump, a tank, a gas tank, a compressor, a piston, a liquid column, a miniature valve, a peristaltic pump, and a pinch valve.

The active actuation means may be applied on the at least one membrane.

In a second aspect, the invention relates to a method for analyzing a seminal sample using a device, the method comprising: introducing the seminal sample into the device, guiding the seminal sample through the device, segregating sperm cells from the seminal sample into a fluid medium, and recovering sperm cells from the fluidic medium.

In one embodiment, introducing the seminal sample into the device further may comprise introducing at least one fluid into the device through at least one inlet. Moreover, introducing at least one fluid into the device precedes introducing the seminal sample into the device.

The at least one fluid may comprise the fluidic medium.

In another embodiment, introducing the at least one fluid into the device further may comprise expelling the at least one fluid through at least one outlet.

The method may comprise guiding the seminal sample through the device comprising the seminal sample through at least one selection channel comprised by the device.

The method may comprise supplying a flow of the at least one fluid in a medium flow direction, wherein the medium flow direction may be also defined by an initial flow point and a terminal flow point.

The method may comprise introducing the seminal sample into the device at the terminal flow point of the medium flow direction.

The method may comprise recovering sperm cell from the fluidic medium at the initial flow point of the medium flow direction.

The method further may comprise establishing a sorting threshold comprising a sperm profile, wherein the method may comprise sorting the sperm cells based on the sorting threshold, wherein the sorting of sperm cells may be performed using a sorting component.

The method further may comprise analyzing sperm cells in the seminal sample using an analyzing component, wherein the analyzing component may be according to any of the preceding system embodiments.

The device may comprise an inlet reservoir, wherein the method may comprise holding the seminal sample in the inlet reservoir.

The seminal sample may comprise a raw-sperm sample.

The device may comprise an outlet reservoir, wherein the method may comprise collecting the sperm sample in the outlet reservoir.

The method may comprise separating the sperm sample from the raw sperm sample, wherein separating the sperm sample from the raw sperm sample precedes collecting the sperm sample in the outlet reservoir.

The method may comprise establishing a direct fluid connection between the inlet reservoir and at least one of: the at least one inlet

The method may comprise establishing a direct fluid connection between the outlet reservoir and at least one of: the least one outlet.

The method may comprise providing a fluid connection between the inlet reservoir and the outlet reservoir.

The fluid connection between the inlet reservoir and the outlet reservoir may comprise a fluid connection passing through the at least one selection channel.

The method using a detection zone comprised by the device, and allowing analysis of the separated seminal sample by means of an analytical device.

The method may comprise arranging at least one separation wall between the detection zone and the analytical device.

The at least one separation wall may comprise a transparent material.

The method may comprise passing a light beam through at least a portion of the separated sperm sample in the detection zone into an optical device.

The method may comprise allocating a known volume of the seminal sample to count the number of sperm cells in the detection zone.

The method may comprise using at least one reference comprised by the detection zone, wherein the at least one reference may comprise one of: an object of known dimensions, and a position.

The method may comprise using the at least one reference to perform at least one of: scaling at one of: the at least one captured image data, and registering at least one of: the at least one captured image data.

The method may comprise impeding helical locomotion of a subpopulation of sperm.

The method may comprise facilitating 2D slither swimming of a slither capable subset of sperm within the at least one selection channel.

The method may comprise simulating natural conditions of a female body via at least one of: a high-viscosity, and physiological buffer in a micro-network.

The method may comprise allocating in the at least one selection channel a volume of at least 0.01 mL, preferably at least 0.02 mL, more preferably at least 0.05 mL and less than 1 mL, preferably less than 0.8 mL, more preferably less than 0.5 mL.

The method may comprise allocating in the at least one selection channel a volume of raw sperm sample comprising a concentration of least 10 million sperm per mL, preferably at least 50 million sperm per mL, more preferably at least 100 million sperm per mL, and less than least 250 million sperm per mL, preferably less than 100 million sperm per mL, more preferably less than 150 million sperm per mL.

The method may comprise supporting and/or preventing a collapsing of the at least at least one selection.

The method may comprise prompting at least one of: at least one valve comprised by the device to assume at least one of: two operational states.

The method may comprise regulating the fluidic connection between the device and the macrofluidic chamber.

The method may comprise prompting the at least one valve to assume a first operational state and a second operational state, wherein the first operational state may be different from the second operational state.

The method may comprise establishing the fluid connection between the device and the microfluidic chamber while the at least one valve may be in the first operational state.

The method may comprise discontinuing the fluid connection between the device and the microfluid chamber while the at least one valve may be in the second operational state.

The method further may comprise regulating a flow withing the device.

The method may comprise facilitating flow of fluids in only one direction through a conduit comprised by the device.

The method may comprise individually and/or independently selecting activation at least one of: the at least two operational state.

The method may comprise actuating an actuation element.

The actuation element may comprise a passive actuation means, wherein the method may comprise facilitating flow of the seminal sample by means of the passive actuation means.

The method may comprise applying the active actuation means via at least one of: a pump, a tank, a gas tank, a compressor, a piston, a liquid column, a miniature valve, a peristaltic pump, and a pinch valve.

The method may comprise applying the active actuation means on the at least one membrane.

In a third aspect, the invention relates to a system for analyzing seminal image data, the system comprising: a detecting component configured to detect sperm cells in the seminal sample, an analyzing component configured to analyze sperm cells in the seminal sample, a sorting component configured to sort sperm cells in the seminal sample, and at least one deep learning component.

The at least one deep learning component may be configured to use seminal image data to generate at least one finding comprising at least one of: a diagnose finding, and a prognose finding.

The at least one finding may comprise information regarding the fertility status of at least one of: a subject, and a course of treatment of the subject.

The treatment may comprise at least one treatment approach to treat infertility in the subject.

The seminal image data may comprise raw image data from the seminal samples.

The seminal image data may comprise processed imaged data obtained by processing the raw image data.

The seminal sample data may comprise at least one calculated value of at least one of: sperm count, sperm morphology, sperm motility, and sperm DNA integrity.

The system may be configured to input data from seminal samples from subjects.

The system may be configured to output data from seminal samples from at least one subject, wherein the outputted data may comprise at least one analytical finding related to one of: the at least one subject.

The system may be configured to train itself to represent features defined by the image data.

The system may be configured to create a map of the outputted data, wherein the map may comprise finding probabilities.

The system may be provided with the seminal sample data and an associated finding.

The system may be configured to autonomously identify at least one feature in the seminal sample data.

The data from subjects with known analytical finding may be used to train the at least one deep learning component.

The system may be configured to enter data from seminal samples from subjects for whom analytical finding may be unknown into the at least one deep learning component.

The system may be configured to identify predictive features in the test data, locate the features on the map of finding probabilities, and/or provide a diagnosis and/or prognosis for the test subjects.

The system may comprise at least one computing component. The system may comprise a semen-analysis component. In on embodiment, at least one of: the at least one computing component comprise a processor unit coupled to at least one of: a tangible, non-transitory memory device, and at least one input/output device.

The system comprise at least one processor coupled to a memory subsystem comprising at least one of: memory device.

Each of the components of the system may be configured bidirectional communication over a network, wherein the network may be at least one of: wired, and wireless.

Each of the components of the system may comprise at least one of: a local component, and a remote component.

The system may be configured to at least one of: receive training data, and obtain training data, wherein the training data may comprise at least one of: seminal sample data, image data, and finding data.

The system may be configured to: use the memory to store the received training data, train the at least one deep learning component.

The memory subsystem may comprise at least one memory device.

The at least one computing component may comprise at least one of: a non-transitory memory device such as a solid-state drive, flash drive, disk drive, hard drive, a subscriber identity module (SIM) card, a secure digital card (SD card), a micro SD card, a solid-state drive (SSD), an optical media, and a magnetic media.

The system may be configured to produce a report and provide the report to a user via an input/output device.

The system may comprise an input/output device comprising a mechanism for transferring data into or out of a computer comprising at least one of: include a video display unit, a printing unit, an alphanumeric input device, a cursor control device, a disk drive unit, a speaker, a touchscreen, an accelerometer, a microphone, a cellular radio frequency antenna, and a network interface device.

The system may be configured to: receive at least one image data of seminal samples and known analytical findings, identify at least one feature within the image data in an unsupervised manner, and create a map of finding probabilities based on the at least one feature.

The system may be configured to: receive image data of seminal samples from a test subject, identify within the at least one image data at least one predictive feature learned from training steps, and locate the at least one predictive feature on the map of finding probabilities to provide at least one of: prognosis, and diagnosis.

The system may be configured for deep learning by a neural network comprising learning multiple levels of representations corresponding to different levels of abstraction and levels form a hierarchy of concepts.

The system may comprise at least one the neural network comprising at least 5 hidden layers, preferably at least 10 hidden layers, more preferably at least 20 hidden layers.

In a fourth aspect, the invention relates to an analytical method for evaluating male fertility using the system as recited herein, the method comprising analyzing seminal sample.

The analytical method may comprise training data comprising seminal sample data.

The analytical method may comprise training data comprising finding data, wherein the finding data may comprise at least one known fertility statuses.

The analytical method may comprise detecting at least one feature within the training seminal sample data by means of the at least one deep learning component.

The analytical method may comprise using a test data comprising seminal sample data from a test subject.

The analytical method may comprise detecting the at least one feature by means of the at least one deep learning component, wherein the at least one feature may be detected in test seminal sample data.

The analytical method may comprise correlating the at least one feature with at least one analytical finding.

The at least one analytical finding may comprise a fertility status.

The analytical method may comprise generating at least one analytical finding for the test subject.

The at least one analytical finding for the test subject may comprise a likelihood of conceive ability.

The at least one analytical finding may comprise at least one of: treatment histories, and results obtained from various types of treatment.

The at least one analytical finding may comprise information related to at least one of: age, medications, consumption of alcohol, consumption of tobacco, consumption of recreational drugs diet, and consumption of dietary supplements including vitamin physical activity levels including exercise and fitness regimens and periods of sitting, frequency of ejaculation, frequency and timing of intercourse including the temporal relationship between intercourse and other factors, clothing and undergarments, use of hot tubs, baths, saunas and other environments exposing testicles to non-physiological temperatures, surgeries including vasectomies and vasectomy reversals, hernia repairs, correction of undescended testicles, treatment of varicoceles, prostate surgery, and other testicular or abdominal procedures, mental and psychological factors including stress levels, exposure to environmental hazards such as industrial chemicals e.g. benzene, toluene, xylene, pesticides, herbicides, other organic solvents, painting materials, and the like, heavy metals or radiation, number of fertilized eggs produced using subject's sperm, number of eggs to which the subject's sperm was exposed, method of introducing the sperm to intrauterine delivery, preparation of the sperm sample, in vitro fertilization (IVF), intracytoplasmic sperm injection (ICSI), number of fertilized eggs implanted, number of fetuses observed at various gestational time points, number of live births, and health status of the fetus or the newborn.

The analytical method providing a numerical representation of objects comprising the at least one feature, wherein the method may comprise using the numerical representation of objects for processing and analyzing at least one of: the at least one image data, and the at least one finding.

The analytical method may comprise combining the vector may often with weights using a dot product in order to construct a linear predictor function.

The analytical method may comprise determining a score for making a prediction based upon the liner predictor function.

The analytical method connecting at least one node in at least one layer.

The at least one node may be connected in the at least one layer, wherein signals travel from an input layer to an output layer.

Each of the at least one node corresponds to a respective to at least one training data patch.

The at least one node may be comprised by at least one hidden layer, wherein the analytical method may comprise calculating the at least one hidden layer as a function of a bias term and a weighted sum of the at least one nodes of the input layer.

The analytical method may comprise assigning a weight each connection between the at least one node of the input layer and the at least one node in the at least one hidden layer.

The analytical method may comprise connecting at least one node in at least one layer.

The layer bias term and the weights between the input layer and the hidden layer may be autonomously learned in the training of the neural network.

The analytical method may comprise generating at least one of: a threshold function, and a limiting function, on at least one of: each connection, and the system.

The analytical method may comprise at least one signal, wherein the at list one signal may be required to surpass at least one of: the at least one threshold function and limiting function before propagating according to a propagation.

The analytical method may comprise using the propagation to perform at least one of: modifying connection weights via means of a forward stimulation, and training the neural network using known correct findings.

The analytical method may comprise associating the at least one feature with the at least one finding to generate an association between the at least one feature and the at least one finding.

The analytical method may comprise representing the association as at least one finding probabilities map, wherein the at least one finding probabilities map a likelihood of a known finding.

The analytical method may comprise correlating may comprise locating at least one features detected in the test seminal sample data on the at least one finding probabilities map.

The analytical method may comprise providing for a test subject from which the seminal sample was obtained at least one of: a prognosis, and a diagnosis.

The at least one finding probabilities map may comprise a likelihood of a given finding for an individual to at least one data point in a seminal sample from the individual.

The at least one finding probabilities map may be established based on the at least one feature identified in the training data as associated with a plurality of findings.

The at least one finding probabilities map may be created autonomously by system.

The at least one finding probabilities map may be created or at least refined with user input.

The analytical method may comprise generating a report comprising at least one of: diagnosis, and prognosis.

The analytical method may comprise selectively transmitting the report to at least one of: test subjects, and treating physicians. either physically or electronically.

The diagnosis may comprise the test subject fertility status.

The analytical method may comprise expressing the diagnosis as a probability comprising a level of confidence.

The diagnosis may comprise a medical stage of at least one of: a condition, and a disease.

The diagnosis may comprise an identification of an element in the seminal sample data associated with at least one of: a low fertility, and a plausible treatment.

The diagnosis may comprise a recommended treatment regimen

The invention also relates to the use of the system mas recited herein for carrying out the method as recited herein.

The present technology is also defined by the following numbered embodiments.

Below, device embodiments will be discussed. These embodiments are abbreviated by the letter "D" followed by a number. When reference is herein made to a D embodiment, those embodiments are meant.
D1. A device for analyzing a seminal sample, the device comprising a microvolume unit configured to allocate at least one fluid, wherein the microvolume unit comprises
   at least one inlet to receive the at least one fluid,
   at least one outlet configured to expel the at least one fluid, and
   at least one channel arranged on the microvolume unit,
   wherein the at least one channel is configured to guide the at least one fluid through the microvolume unit.
D2. The device according to the preceding embodiment, wherein the device comprises an inlet reservoir configured to hold the seminal sample.
D3. The device according to the preceding embodiment, wherein the seminal sample comprises a raw-sperm sample.
D4. The device according to any of the 2 the preceding embodiments, wherein the device comprises an outlet reservoir for collecting the sperm sample, wherein the sperm sample is separated from the raw sperm sample.
D5. The device according to any of the preceding device embodiments, wherein the at least one channel comprises at least one selection channel arranged between the inlet reservoir and the outlet reservoir.
D6. The device according any of the preceding device embodiments and with features of embodiments D2 and D4, wherein the inlet reservoir is directly fluidic connected to at least one of the least one inlet, and the outlet reservoir is directly fluidic connect to at least one of the least one outlet.
D7. The device according to any of the preceding device embodiments, wherein the at least one selection channel is configured to provide a fluid connection between the inlet reservoir and the outlet reservoir.
D8. The device according to any of the preceding device embodiments, wherein the device comprises a detection zone configured to allow the analysis of the separated seminal sample by means of the optical analytical device.
D9. The device according to any of the preceding device embodiments, wherein the device comprises at least one separation wall is arranged between the detection zone and the analytical device.
D10. The device according to any of the preceding device embodiments, wherein the at least one separation wall comprises a transparent material configured to allow the at least light source passing through a portion of the separated sperm sample in the detection zone into the optical device.
D11. The device according to any of the preceding device embodiments, wherein the valve detection zone is configured to allocate a known volume of the seminal sample to count the number of sperm cells in the portion of the detection zone.
D12. The device according to any of the preceding device embodiments, wherein the detection zone comprises at least one reference comprising one of: an object of known dimensions, and a position.
D13. The device according to the preceding embodiment, wherein the at least one reference is configured to allow at least one of: scaling at one of the at least one captured image data, and registering at least one of the at least one captured image data.
D14. The device according to the preceding embodiments, wherein the geometry is configured to allow 2D slither swimming of a slither capable subset of sperm within the at least one selection channel.
D15. The device according to any of the preceding device embodiments, wherein the device is further configured to simulate natural conditions of a female body via at least one of: a high-viscosity, and physiological buffer in a micro-network
D16. The device according to any of the preceding device embodiments and with features of embodiment D5, wherein the at least one selection channel comprises a height sufficiently low to restrict the helical locomotion of sperm within the at least one selection channel.
D17. The device according to the preceding embodiment, wherein the at least one selection channel comprises a channel height lower than 10µm, preferably lower than 5µm, more preferably lower than 3µm, such as 2µm.
D18. The device according to any of the preceding device embodiments and with features of embodiment D5, wherein the at least one selection channel comprises a channel width between 10 µm and 100 µm, preferably between 25 µm and 150 µm, more preferably between 50 µm and 100 µm.
D19. The device according to any of the preceding device embodiments and with features of embodiment D5, wherein the at least one selection channel a channel length between 1 and 10 cm, preferably between 3 and 8 cm, such as between 5 and 7 cm.
D20. The device according to any of the preceding device embodiments and with features of embodiment D5, wherein the at least one selection channel is configured to allocate a volume of at least 0.01 mL, preferably at least 0.02 mL, more preferably at least 0.05 mL and less than 1 mL, preferably less than 0.8 mL, more preferably less than 0.5 mL.
D21. The device according to any of the preceding device embodiments and with features of embodiment D5, wherein the at least one selection channel is configured to allocate a volume of raw sperm sample comprising a concentration of least 10 million sperm per mL, preferably at least 50 million sperm per mL, more preferably at least 100 million sperm per mL, and less than least 250 million sperm per mL, preferably less than 100 million sperm per mL, more preferably less than 150 million sperm per mL.
D22. The device according to any of the preceding device embodiments and with features of embodiment D5, wherein the at least one selection channel comprises a single selection channel.
D23. The device according to the preceding, wherein the single selection channel comprises a ratio between the channel width and channel height of at least 2, preferably at least 4, more preferably at least 5.
D24. The device according to any of the preceding embodiment and with features of embodiment D5, wherein the at least one selection channel comprises at least one support structures arranged longitudinal along the channel length.
D25. The device according to the preceding embodiments, wherein the at least one support structure is configured to support and/or prevent a collapsing of the at least one single selection channel.
D26. The device according to any of the preceding device embodiments, wherein the device comprises a hard material comprising at least one of: silicon, and an elastomeric material such as silicone rubber.
D27. The device according to any of the preceding device embodiments, wherein the device comprises a semi conductive material.
D28. The device according the preceding embodiment, wherein the semi conductive material comprises at least one of: polysilicon, silicon nitride, and silicon dioxide.
D29. The device according to any of the preceding device embodiments, wherein the device comprises a metal and/or a metallic alloy.
D30. The device according to any of the preceding device embodiments, wherein the metal and/or metallic alloy is at least one of: magnetic, and electrically conductive.
D31. The device according to any of the preceding device embodiments, wherein the device comprises at least one of: polydimethylsiloxane (PDMS), poly(methyl methacrylate) (PMMA), a suitable thermoplastic such as polycarbonates, polystyrene, and glass.
D32. The device according to any of the preceding device embodiments, wherein the device comprises at least one valve configured to assume at least two operational states.
D33. The device according to the preceding embodiment, wherein the at least one valve is configured to regulate the fluidic connection between the device and the macrofluidic chamber.
D34. The device according to the 2 preceding embodiments, wherein the at least one valve is configured to assume a first operational state and a second operational state, wherein the first operational state and different from the second operational state, wherein the at least one valve is configured to
   establish the fluid connection between the device and the microfluidic chamber while in the first operational state,
   discontinue the fluid connection between the device and the microfluid chamber while in the second operational state.
D35. The device according to any of the 3 preceding embodiments, wherein the at least one valve component comprises at least one internal valve configured to regulate a flow within the device.
D36. The device according to any of the 4 preceding embodiments, wherein at least one of the at least one valve is a check valve configured to allow flow of fluids in only one direction through a conduit comprised by the check valve
D37. The device according to any of the preceding 5 embodiments and with features of embodiment D34, wherein the at least one valve is configured to individually and/or independently be selected to assume at least one of the at least two operational state.
D38. The device according to any of the preceding device embodiments, wherein the device comprises an actuation element.
D39. The device according to the preceding embodiment, wherein the actuation element comprises an active actuation means comprising at least one of: air pressure, electrostatic forces, and magnetic forces.
D40. The device according to any of the 2 preceding embodiments, wherein the actuation element comprises a passive actuation means comprising means to facilitate flow of the seminal sample.
D41. The device according to the preceding embodiment, wherein the passive actuation means comprises a viscosity-altering agent comprising at least one of: a liquefying agent, and a surfactant.
D42. The device according to any of the preceding device, wherein the device comprises at least one membrane.
D43. The device according to any of the preceding device embodiments and with features of embodiment D39, wherein the active actuation means is applied from at least one of: a pump, a tank, a gas tank, a compressor, a piston, a liquid column, a miniature valve, a peristaltic pump, and a pinch valve.
D45. The device according to any of the preceding device embodiments and with features of embodiments D39 and D42, wherein the active actuation means is applied on the at least one membrane.
Below, method embodiments will be discussed. These embodiments are abbreviated by the letter "N" followed by a number. When reference is herein made to a N embodiment, those embodiments are meant.
N1. A method for analyzing a seminal sample using a device, the method comprising introducing the seminal sample into the device,
   guiding the seminal sample through the device,
   segregating sperm cells from the seminal sample into a fluid medium, and
   recovering sperm cells from the fluidic medium.
N2. The method according to the preceding embodiment, wherein the device comprises any of the features according to any of the device embodiments.
N3. The method according to any of the 2 preceding embodiments, wherein introducing the seminal sample into the device further comprises introducing at least one fluid into the device through at least one inlet.
N4. The method according to the preceding embodiment, wherein introducing at least one fluid into the device precedes introducing the seminal sample into the device.
N5. The method according to the 2 preceding embodiments, wherein the at least one fluid comprises the fluidic medium.
N6. The method according to the 3 preceding embodiments, wherein introducing the at least one fluid into the device further comprises expelling the at least one fluid through at least one outlet.
N7. The method according to any of the preceding method embodiments, wherein guiding the seminal sample through the device comprising the seminal sample through at least one selection channel comprised by the device.
N8. The method according to any of the preceding method embodiments, wherein the method comprises supplying a flow of the at least one fluid in a medium flow direction, wherein the medium flow direction is also defined by an initial flow point and a terminal flow point.
N9. The method according to any of the preceding method embodiments, wherein the method comprises introducing the seminal sample into the device at the terminal flow point of the medium flow direction.
N10. The method according to any of the preceding method embodiments, wherein the method comprises recovering sperm cell from the fluidic medium at the initial flow point of the medium flow direction.
N11. The method according to any of the preceding method embodiments, wherein the method further comprises establishing a sorting threshold comprising a sperm profile, wherein the method comprises sorting the sperm cells based on the sorting threshold.
N12. The method according to the preceding embodiment, wherein the sorting of sperm cells is performed using a sorting component.
N13. The method according to any of the preceding method embodiments, wherein the method further comprises analyzing sperm cells in the seminal sample using an analyzing component.
N14. The method according to the preceding embodiment, wherein the analyzing component is according to any of the preceding system embodiments.
N15. The method according to any of the preceding method embodiment, wherein the device comprises an inlet reservoir, wherein the method comprises holding the seminal sample in the inlet reservoir.
N16. The method according to the preceding embodiment, wherein the seminal sample comprises a raw-sperm sample.
N17. The method according to any of the 2 the preceding embodiments, wherein the device comprises an outlet reservoir, wherein the method comprises collecting the sperm sample in the outlet reservoir.
N18. The method according to the preceding embodiment, wherein the method comprises separating the sperm sample from the raw sperm sample.
N19. The method according to the 2 preceding embodiments, wherein separation the sperm sample from the raw sperm sample precedes collecting the sperm sample in the outlet reservoir.
N20. The method according to any of the preceding method embodiments, wherein the method comprises establishing a direct fluid connection between the inlet reservoir and at least one of the at least one inlet
N21. The method according to any of the preceding method embodiments, wherein the method comprises establishing a direct fluid connection between the outlet reservoir and at least one of the least one outlet.
N22. The method according to any of the preceding method embodiments, wherein the method comprises providing a fluid connection between the inlet reservoir and the outlet reservoir.
N23. The method according to the preceding embodiment, wherein the fluid connection between the inlet reservoir and the outlet reservoir comprises a fluid connection passing through the at least one selection channel.
N24. The method according to any of the preceding method embodiments, wherein the method
   using a detection zone comprised by the device, and
   allowing analysis of the separated seminal sample by means of an analytical device.
N25. The method according to any of the preceding method embodiments, wherein the method comprises arranging at least one separation wall between the detection zone and the analytical device.
N26. The method according to the preceding method embodiment, wherein the at least one separation wall comprises a transparent material.
N27. The method according to the 2 preceding embodiments, wherein the method comprises passing a light beam through at least a portion of the separated sperm sample in the detection zone into an optical device.
N28. The method according to any of the preceding method embodiments, wherein the method comprises allocating a known volume of the seminal sample to count the number of sperm cells in the detection zone.
N29. The method according to any of the preceding method embodiments, wherein the method comprises using at least one reference comprised by the detection zone, wherein the at least one reference comprises one of: an object of known dimensions, and a position.
N30. The method according to the preceding embodiment, wherein the method comprises using the at least one reference to perform at least one of
   scaling at one of the at least one captured image data, and
   registering at least one of the at least one captured image data.
N31. The method according to any of the preceding method embodiments, wherein the method comprises impeding helical locomotion of a subpopulation of sperm.
N32. The method according to the preceding embodiments, where the method comprises facilitating 2D slither swimming of a slither capable subset of sperm within the at least one selection channel.
N33. The method according to any of the preceding method embodiments, wherein the method comprises simulating natural conditions of a female body via at least one of: a high-viscosity, and physiological buffer in a micro-network.
N34. The method according to any of the preceding method embodiments, wherein the method comprises allocating in the at least one selection channel a volume of at least 0.01 mL, preferably at least 0.02 mL, more preferably at least 0.05 mL and less than 1 mL, preferably less than 0.8 mL, more preferably less than 0.5 mL.
N35. The method according to any of the preceding method embodiments, wherein the method comprises allocating in the at least one selection channel a volume of raw sperm sample comprising a concentration of least 10 million sperm per mL, preferably at least 50 million sperm per mL, more preferably at least 100 million sperm per mL, and less than least 250 million sperm per mL, preferably less than 100 million sperm per mL, more preferably less than 150 million sperm per mL.
N36. The method according to any of the preceding embodiment and with features of embodiment xx, wherein the method comprises supporting and/or preventing a collapsing of the at least at least one selection.
N37. The method according to any of the preceding method embodiments, wherein the method comprises prompting at least one of at least one valve comprised by the device to assume at least one of two operational states.
N38. The method according to the preceding embodiment, wherein the method comprises regulating the fluidic connection between the device and the macrofluidic chamber.
N39. The method according to the 2 preceding embodiments, wherein the method comprises prompting the at least one valve to assume a first operational state and a second operational state, wherein the first operational state is different from the second operational state.
N40. The method according to the 3 preceding embodiments, wherein the method comprises establishing the fluid connection between the device and the microfluidic chamber while the at least one valve is in the first operational state.
N41. The method according to any of the 4 preceding embodiments, wherein the method comprises discontinuing the fluid connection between the device and the microfluid chamber while the at least one valve is in the second operational state.
N42. The method according to any of the 3 preceding embodiments, wherein the method further comprises regulating a flow withing the device.
N43. The method according to any of the 4 preceding embodiments, wherein the method comprises facilitating flow of fluids in only one direction through a conduit comprised by the device.
N44. The method according to any of the preceding 5 embodiments, wherein the method comprises individually and/or independently selecting activation at least one of the at least two operational state.
N45. The method according to any of the preceding method embodiments, wherein the method comprises actuating an actuation element.
N46. The method according to the preceding embodiment, wherein the actuation element comprises an active actuation means comprising at least one of: air pressure, electrostatic forces, and magnetic forces.
N47. The method according to any of the 2 preceding embodiments, wherein the actuation element comprises a passive actuation means, wherein the method comprises facilitating flow of the seminal sample by means of the passive actuation means.
N48. The method according to the preceding embodiment, wherein the passive actuation means comprises a viscosity-altering agent comprising at least one of: a liquefying agent, and a surfactant.
N49. The method according to any of the preceding device, wherein the device comprises at least one membrane, wherein the method comprises applying the active actuation means via at least one of: a pump, a tank, a gas tank, a compressor, a piston, a liquid column, a miniature valve, a peristaltic pump, and a pinch valve.
N50. The method according to any of the preceding method, wherein the method comprises applying the active actuation means on the at least one membrane.

Below, system embodiments will be discussed. These embodiments are abbreviated by the letter "A" followed by a number. When reference is herein made to an A embodiment, those embodiments are meant.
A1. A system for analyzing seminal image data, the system comprising
   a detecting component configured to detect sperm cells in the seminal sample,
   an analyzing component configured to analyze sperm cells in the seminal sample,
   a sorting component configured to sort sperm cells in the seminal sample, and
   at least one deep learning component.
A2. The system according to the preceding embodiment, wherein the at least one deep learning component is configured to use seminal image data to generate at least one finding comprising at least one of: a diagnose finding, and a prognose finding.
A3. The system according to the preceding embodiment, wherein the at least one finding comprises information regarding the fertility status of at least one of: a subject, and a course of treatment of the subject.
A4. The system according to the preceding embodiment, wherein the treatment comprises at least one treatment approach to treat infertility in the subject.
A5. The system according to any of the preceding system embodiments, wherein the seminal image data comprises raw image data from the seminal samples.
A6. The system according to any of the preceding system embodiments, wherein the seminal image data comprises processed imaged data obtained by processing the raw image data.
A7. The system according to any of the preceding system embodiments, wherein the seminal sample data comprises at least one calculated value of at least one of: sperm count, sperm morphology, sperm motility, and sperm DNA integrity.
A8. The system according to any of the preceding system embodiments, wherein the system is configured to input data from seminal samples from subjects.
A9. The system according to any of the preceding system embodiments, wherein the system is configured to output data from seminal samples from at least one subject, wherein the outputted data comprises at least one analytical finding related to one of the at least one subject.
A10. The system according to any of the preceding system embodiments, wherein the system is configured to train itself to represent features defined by the image data.
A11. The system according to any of the preceding system embodiments, wherein the system is configured to create a map of the outputted data, wherein the map comprises finding probabilities.
A12. The system according to any of the preceding system embodiments, wherein the system is provided with the seminal sample data and an associated finding.
A13. The system according to any of the preceding system embodiments, wherein the system is configured to autonomously identify at least one feature in the seminal sample data.
A14. The system according to any of the preceding system embodiments, wherein the data from subjects with known analytical finding is used to train the at least one deep learning component.
A15. The system according to any of the preceding system embodiments, the system is configured to enter data from seminal samples from subjects for whom analytical finding are unknown into the at least one deep learning component.
A16. The system according to any of the preceding system embodiments, the system is configured to identify predictive features in the test data, locate the features on the map of finding probabilities, and/or provide a diagnosis and/or prognosis for the test subjects.
A17. The system according to any of the preceding system embodiments, the system comprises at least one computing component.
A18. The system according to any of the preceding system embodiments, the system comprises a semen-analysis component.
A19. The system according to embodiment A17, wherein at least one of the at least one computing component comprise a processor unit coupled to at least one of: a tangible, non-transitory memory device, and at least one input/output device.
A20. The system according to any of the preceding system embodiments, the system comprise at least one processor coupled to a memory subsystem comprising at least one of memory device.
A21. The system according to any of the preceding system embodiments, wherein each of the components of the system is configured bidirectional communication over a network, wherein the network is at least one of: wired, and wireless.
A22. The system according to the preceding embodiment, where each of the components of the system comprises at least one of: a local component, and a remote component.
A23. The system according to any of the preceding system embodiments, the system is configured to at least one of: receive training data, and obtain training data, wherein the training data comprises at least one of: seminal sample data, image data, and finding data.
A24. The system according to any of the preceding system embodiments, the system is configured to use the memory to: store the received training data, train the at least one deep learning component.
A25. The system according to any of the preceding system embodiments and with features of A20, wherein the memory subsystem comprises at least one of memory device.
A26. The system according to any of the preceding system embodiments and with features of A17, wherein the at least one computing component comprises at least one of: a non-transitory memory device such as a solid-state drive, flash drive, disk drive, hard drive, a subscriber identity module (SIM) card, a secure digital card (SD card), a micro SD card, a solid-state drive (SSD), an optical media, and a magnetic media.
A27. The system according to any of the preceding system embodiments, wherein the system is configured to produce a report and provide the report to a user via an input/output device.
A28. The system according to any of the preceding system embodiments, wherein the system comprises an input/output device comprising a mechanism for transferring data into or out of a computer comprising at least one of: include a video display unit, a printing unit, an alphanumeric input device, a cursor control device, a disk drive unit, a speaker, a touchscreen, an accelerometer, a microphone, a cellular radio frequency antenna, and a network interface device.
A29. The system according to any of the preceding system embodiments, wherein the system is configured to
   receive at least one image data of seminal samples and known analytical findings, identify at least one feature within the image data in an unsupervised manner, and create a map of finding probabilities based on the at least one feature.
A30. The system according to any of the preceding system embodiments, wherein the system is configured to
   receive image data of seminal samples from a test subject,
   identify within the at least one image data at least one predictive feature learned from training steps, and
   locate the at least one predictive feature on the map of finding probabilities to provide at least one of: prognosis, and diagnosis.
A31. The system according to any of the preceding system embodiments, the system is configured for deep learning by a neural network comprising learning multiple levels of representations corresponding to different levels of abstraction and levels form a hierarchy of concepts.
A32. The system according to any of the preceding system embodiments, wherein the system comprises at least one the neural network comprising at least 5 hidden layers, preferably at least 10 hidden layers, more preferably at least 20 hidden layers.

Below, analytical method embodiments will be discussed. These embodiments are abbreviated by the letter "P" followed by a number. When reference is herein made to a P embodiment, those embodiments are meant.
P1. An analytical method for evaluating male fertility using a system according to any of the preceding system embodiments, the method comprising analyzing seminal sample.
P2. The analytical method according to the preceding embodiment, wherein the analytical method comprises training data comprising seminal sample data.
P3. The analytical method according to any of the preceding analytical method embodiments, wherein the analytical method comprises training data comprising finding data, wherein the finding data comprises at least one known fertility statuses.
P4. The analytical method according to any of the preceding analytical method embodiments, wherein the analytical method comprises detecting at least one feature within the training seminal sample data by means of the at least one deep learning component.
P5. The analytical method according to any of the preceding analytical method embodiments, wherein the analytical method comprises using a test data comprising seminal sample data from a test subject.
P6. The analytical method according to any of the preceding analytical method embodiments, wherein the analytical method comprises detecting the at least one feature by means of the at least one deep learning component, wherein the at least one feature is detected in test seminal sample data.
P7. The analytical method according to the preceding embodiment, wherein the analytical method comprises correlating the at least one feature with at least one analytical finding.
P8. The analytical method according to any of the 2 preceding embodiments, wherein the at least one analytical finding comprises a fertility status.
P9. The analytical method according to any of the 3 preceding embodiments, wherein the method comprises generating at least one analytical finding for the test subject.
P10. The analytical method according to the preceding embodiment, wherein the at least one analytical finding for the test subject comprises a likelihood of conceive ability.
P11. The analytical method according to any of the preceding analytical method embodiments, wherein the at least one analytical finding comprises at least one of: treatment histories, and results obtained from various types of treatment.
P12. The analytical method according to any of the preceding analytical method embodiments, wherein the at least one analytical finding comprises information related to at least one of: Age, medications, consumption of alcohol, consumption of tobacco, consumption of recreational drugs diet, and consumption of dietary supplements including vitamins, physical activity levels including exercise and fitness regimens and periods of sitting, frequency of ejaculation, frequency and timing of intercourse including the temporal relationship between intercourse and other factors, clothing and undergarments, use of hot tubs, baths, saunas and other environments exposing testicles to non-physiological temperatures, surgeries including vasectomies and vasectomy reversals, hernia repairs, correction of undescended testicles, treatment of varicoceles, prostate surgery, and other testicular or abdominal procedures, mental and psychological factors including stress levels, exposure to environmental hazards such as industrial chemicals e.g. benzene, toluene, xylene, pesticides, herbicides, other organic solvents, painting materials, and the like, heavy metals or radiation, number of fertilized eggs produced using subject's sperm, number of eggs to which the subject's sperm was exposed, method of introducing the sperm to intrauterine delivery, preparation of the sperm sample, in vitro fertilization (IVF), intracytoplasmic sperm injection (ICSI), number of fertilized eggs implanted, number of fetuses observed at various gestational time points, number of live births, and health status of the fetus or the newborn.
P13. The analytical method according to any of the preceding analytical method embodiments, wherein the analytical method providing a numerical representation of objects comprising the at least one feature, wherein the method comprises using the numerical representation of objects for processing and analyzing at least one of: the at least one image data, and the at least one finding.
P14. The analytical method according to any of the preceding analytical method embodiments, wherein the analytical method comprises combining the vector may often with weights using a dot product in order to construct a linear predictor function.
P15. The analytical method according to the preceding embodiment, wherein the analytical method comprises determining a score for making a prediction based upon the liner predictor function.
P16. The analytical method according to any of the preceding analytical method embodiments, wherein the analytical method connecting at least one node in at least one layer.
P17. The analytical method according to the preceding embodiment, wherein the at least one node is connected in the at least one layer, wherein signals travel from an input layer to an output layer.
P18. The analytical method according to any of the 2 preceding embodiments, wherein each of the at least one node corresponds to a respective to at least one training data patch.
P19. The analytical method according to any of the preceding analytical method embodiments, wherein the at least one node is comprised by at least one hidden layer, wherein the analytical method comprises calculating the at least one hidden layer as a function of a bias term and a weighted sum of the at least one nodes of the input layer.
P20. The analytical method according to the preceding embodiment, wherein the method comprises assigning a weight each connection between the at least one node of the input layer and the at least one node in the at least one hidden layer.
P21. The analytical method according to any of the preceding analytical method embodiments, wherein the analytical method comprises connecting at least one node in at least one layer.
P22. The analytical method according to any of the preceding analytical method embodiments, wherein the layer bias term and the weights between the input layer and the hidden layer are autonomously learned in the training of the neural network.
P23. The analytical method according to any of the preceding analytical method embodiments, wherein the analytical method comprises generating at least one of: a threshold function, and a limiting function, on at least one of: each connection, and the system.
P24. The analytical method according to the preceding embodiment, wherein the method comprises at least one signal, wherein the at list one signal is required to surpass at least one of the at least one threshold function and limiting function before propagating according to a propagation.
P25. The analytical method according to any of the 2 preceding embodiments, wherein the method comprises using the propagation to perform at least one of
   modifying connection weights via means of a forward stimulation, and
   training the neural network using known correct findings.
P26. The analytical method according to any of the preceding analytical method embodiments, wherein the analytical method comprises associating the at least one feature with the at least one finding to generate an association between the at least one feature and the at least one finding.
P27. The analytical method according to the preceding embodiment, wherein the analytical method comprises representing the association as at least one finding probabilities map, wherein the at least one finding probabilities map a likelihood of a known finding.
P28. The analytical method according to any of the preceding analytical method embodiments, wherein the analytical method comprises correlating comprises locating at least one features detected in the test seminal sample data on the at least one finding probabilities map.
P29. The analytical method according to any of the preceding analytical method embodiments, wherein the analytical method comprises providing for a test subject from which the seminal sample was obtained at least one of: a prognosis, and a diagnosis.
P30. The analytical method according to any of the preceding analytical method embodiments and with features of embodiment P27, wherein the at least one finding probabilities map comprises a likelihood of a given finding for an individual to at least one data point in a seminal sample from the individual.
P31. The analytical method according to any of the preceding analytical method embodiments and with features of embodiment P27, wherein the at least one finding probabilities map is established based on the at least one feature identified in the training data as associated with a plurality of findings.
P32. The analytical method according to any of the preceding analytical method embodiments and with features of embodiment P27, wherein the at least one finding probabilities map is created autonomously by system.
P33. The analytical method according to any of the preceding analytical method embodiments and with features of embodiment P27, wherein the at least one finding probabilities map is created or at least refined with user input.
P34. The analytical method according to any of the preceding analytical method embodiments, wherein the analytical method comprises generating a report comprising at least one of: the diagnosis, and prognosis.
P35. The analytical method according to any of the preceding analytical method embodiments, wherein the analytical method comprises selectively transmitting the report to at least one of: test subjects, and treating physicians. either physically or electronically.
P36. The analytical method according to any of the preceding analytical method embodiments, wherein the diagnosis comprises the test subject fertility status.
P37. The analytical method according to any of the preceding analytical method embodiments, wherein the analytical method comprises expressing the diagnosis as a probability comprising a level of confidence.
P38. The analytical method according to any of the preceding analytical method embodiments, wherein the diagnosis comprises a medical stage of at least one of: a condition, and a disease.
P39. The analytical method according to any of the preceding analytical method embodiments, wherein the diagnosis comprises an identification of an element in the seminal sample data associated with at least one of: a low fertility, and a plausible treatment.
P40. The analytical method according to any of the preceding analytical method embodiments, wherein the diagnosis comprises a recommended treatment regimen
U1. Use of the system according to any of the preceding system embodiments for carrying out the method according to any of the preceding method embodiments.

The present invention will now be described with reference to the accompanying drawings which illustrate embodiments of the invention. These embodiments should only exemplify, but not limit, the present invention.
- Fig. 1: schematically depicts a microfluidic chip according to embodiments of the present invention;
- Fig. 2: schematically depicts an embodiment of a method of analyzing a semen sample using a processor unit according to embodiments of the present invention;
- Fig. 3: depicts a deep learning component and an embodiment of a method for evaluating fertility according to embodiments of the present invention.

It is noted that not all the drawings carry all the reference signs. Instead, in some of the drawings, some of the reference signs have been omitted for sake of brevity and simplicity of illustration. Embodiments of the present invention will now be described with reference to the accompanying drawings

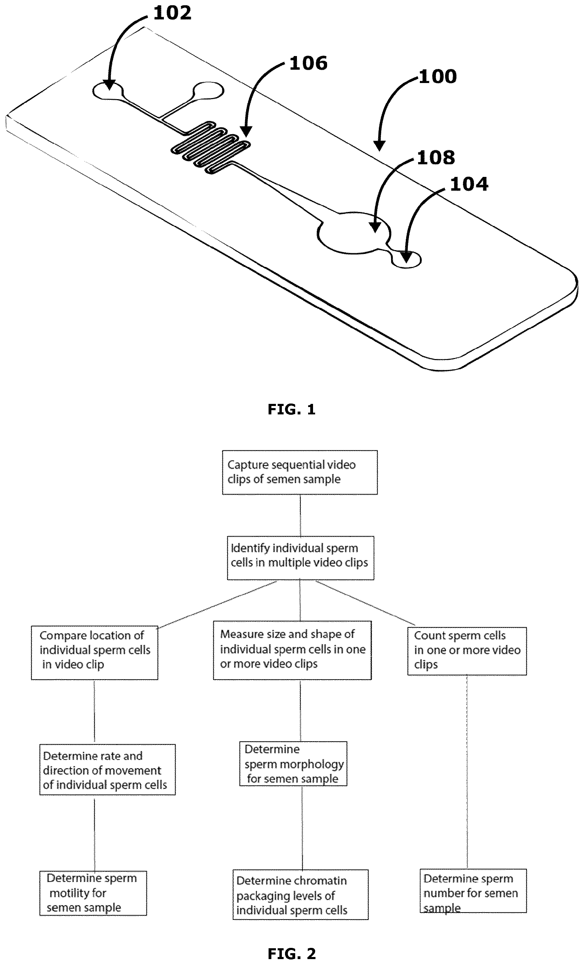

Fig. 1 schematically depicts a microfluidic chip 100 according to embodiments of the present invention. In simple terms, the microfluidic chip 100 may comprise an inlet reservoir 102 configured to hold a raw sperm sample, an outlet reservoir 104 for collecting a sperm sample separated from the raw sperm sample at least one selection channel 106 arranged between the inlet reservoir 102 and the outlet reservoir 104. It should be understood that at least one selection channel 106 is configured to provide a fluid connection between the inlet reservoir 102 and the outlet reservoir 104. Furthermore, it should be understood that the term fluid connection is intended to describe the free movement, in presence or absence of an external source, of fluids from an initial point such as the inlet reservoir 102 in direction to a final point such as the outlet reservoir 104, any intermediated point and/or vice versa.

The microfluidic chip 100 may also be referred to as microfluidic element 100, microfluidic device 100 or simply as microfluidic 100, element 100 and/or chip 100. In one embodiment, the microfluidic element 100 may comprise a detection zone 108 which is configured to allow the analysis of the separated semen sample by means of the analytical device 100. The microfluidic chip 100 may also comprise at least one separation wall (not shown), which may be arranged between the detection zone 108 and the analytical device 100. The at least one separation wall may comprise a transparent material so that a light source can pass through a portion of the separated sperm sample in the detection zone 108 into the optical device 100. It should be understood that the detection zone 108 may allocated a known volume of sample, which may be advantageous, as it may allow to count the number of sperm cells in a given portion of the detection zone 108, which can be extrapolated to determine the sperm number in the raw semen sample. It should also be understood that the term "sperm number of a semen sample" is intended to refer to the number of sperm cells per unit of volume of semen and it may interchangeably be used with sperm count, sperm density or semen concentration In one embodiment, the detection zone 108 may also include one or more reference objects of known dimensions and/or position to allow scaling and registration of images captured by the optical device.

In one embodiment, the at least one selection channel 106 may comprise a geometry configured to impede helical locomotion of a subpopulation of sperm and allow 2D slither swimming of a slither capable subset of sperm within the at least one selection channel 106. It should be understood that the terms "slithering" and "slither swimming" are intended to refer to a manner of sperm locomotion characterized by a two-dimensional movement of the sperm's tail, i.e. sperm flagellum.

Alternatively or additionally, the microfluidic device 100 may be configured to simulate natural conditions of a female body via plurality of properties such as, for example, but not limited to, using a high-viscosity, physiological buffer in a micro-network.

Moreover, the at least one selection channel 106 may comprise a dimension sufficiently low so as it may restrict the helical locomotion of sperm within the at least one selection channel 106. For instance, in one embodiment, the at least one selection channel 106 may comprise a channel dimension between 1.0 vtm to 10.0 vtm.

Furthermore, the at least one selection channel 106 may comprise a channel between 1 and 10 cm, preferably between 3 and 8 cm, such as between 5 and 7 cm. The channel length may be selected to facilitate and/or encourage a slither swimming subset of sperm to traverse the at least one selection channel 106.

In another embodiment, the at least one selection channel 106 may comprise a channel height between 0.5 and 4µm, preferably between 1 and 3µm, such as 2µm.

In one embodiment, each of the at least one selection channel 106 may be configured to allocate a volume of approximately 0.01 mL, so that the raw sperm sample may comprise a concentration of approximately 100 million sperm per mL.

In one embodiment, the at least one selection channel 106 may comprise a channel width between 50 and 400 µm, more preferably between 100 and 300µm, such as 150µm. In another embodiment, the at least one selection channel 106 may comprise a single selection channel 106 comprising a channel width significantly larger than the channel height. The single selection channel 106 may comprise a channel length and may further comprise a plurality of support structures arranged longitudinal along the channel length. The plurality of support structures may be configured to support and/or prevent a collapsing of the single selection channel 106.

Moreover, the microfluidic chip 100 may comprise hard material, such as silicon, and/or elastomeric material, such as silicone rubber. In another embodiment, the microfluidic chip 100 may comprise a semi conductive material, such as polysilicon, silicon nitride, silicon dioxide, and/or a metal which may be magnetic or electrically conductive.

In a further embodiment, the microfluidic chip 100 may comprise at least one of: polydimethylsiloxane (PDMS), poly (methyl methacrylate) (PMMA), suitable thermoplastics such as polycarbonates, polystyrene, and/or glass.

Furthermore, the microfluidic chip 100 may comprise at least one valve configured to assume at least one operational state, such as, for example, an open and a close state, which may allow the at least one valve to regulate the fluidic connection between the microfluidic chip 100 and the macrofluidic fluidic chamber. The at least one valve may further comprise one or more internal valves configured to regulate a flow within the microfluidic element 100. Additionally or alternatively, one of the at least one valve may be a check valve configured to allow flow of fluids, such as semen, in only one direction through a conduit.

In a further embodiment, the at least one valve may be individually addressable, i.e. each of the at least one valve may individually and/or independently be selected to assume at least one of the at least one operational state.

Flow of fluids, such as semen, through the microfluidic element may be actuated by at least one of active means, passive means, or any combination thereof. For example, but not limited to, active means may include application of a fluid pressure such as air pressure, electrostatic forces, and/or magnetic forces. For example, flow of fluids through an elastomeric microfluidic element may be actuated by application of a fluid pressure such as air pressure, to a membrane of the element. Fluid pressure may also be applied from at least one of a pump/tank system, a gas tank, compressor, piston system, liquid column, one or more miniature valves, peristaltic pumps, pinch valves, and other systems known in the art. Passive actuation may comprise means that facilitate flow of the sample, such as adding a viscosity-altering agent to the semen sample within the analytical device. The viscosity-altering agent may be at least one of a liquefying agent, surfactant, soap, detergent. The semen sample and/or an experimental buffer may be loaded into separate syringes and placed in fluid connection to an inlet and an outlet of each channel of the analytical device, respectively. The syringes may comprise at least one of metal syringes, plastic syringes, glass syringes or any combination thereof.

The loaded syringes may act as external reservoirs connected to the inlet and the outlet reservoirs of the microfluidic device. Once the syringes or other form or reservoir are connected to the device, the raw sample and buffer may be injected into the device using one or more suitable pumps with suitable flow rates. For example, the semen sample and/or buffer agent may be injected into the device using two syringe pumps, for instance, set at 1.8 pl min-1 and 2.5 pl min flow rates, respectively

Basal fluid media sample may, for example, comprise a buffering solution of 4-(2-hydroxyethil)-1-piperazineethanesulfonic acid (HEPES), 3-(N-morpholino) propane sulfonic acid (MOPS), 2-[[1,3-dihydroxy-2(hydroxymethyl)propan-2-yl] amino] ethanesulfonic acid (TES) or tris(hydroxymethyl)aminomethane (Tris), or a solution of human tubal fluid. HEPES-buffered solution chemically may comprise 135 mM NaCl, 5 mM KCI, 0.6 mM Na₂HPO₄.xH₂0 wherein is an integer between 0 and 10, 25 mM HEPES and 12 mM D-glucose, which may further be supplemented with 1 mg/mL polyvinyl alcohol, which may be particularly advantageous, as it may allow to prevent sperm from attaching to surfaces, and 0.5% w/w methyl cellulose.

Furthermore, the system may comprise a means for transferring a portion of the semen sample from the macrofluidic fluidic chamber and the microfluidic chip. For example, the system may comprise a pump coupled to the cartridge to pump a portion of the semen sample into the microfluidic chip.

Fig. 2 schematically depicts a method of analyzing a semen sample using a processor unit to determine sperm number, sperm morphology, and sperm motility of a semen sample according to embodiments of the invention.

In a first step, sequential images of a portion of the semen sample may be captured at defined intervals further detailed below. The portion of the semen sample being imaged may be in the detection zone of a microfluidic element of a cartridge, and images may be captured by an optical device that may comprise a lens system 321 and a sensor. The optical data may be sent from a sensor to a processor. The interval between sequentially captured images of the semen sample may be any suitable interval for determining sperm motility. It should be understood for interval the inverse relation of the image capture rate, and either parameter may be used to express the frequency of sequentially captured images. For example, the image capture rate may be about 10 s-1, 20 s-1, 30 s-1, 60 s-1, 90 s-1, 150 s-1, 300 s-1, 600 s-1, or 900 s-1.

In a second, individual sperm cells may be identified in multiple images. Image features such as brightness, color, pixel intensity, and the like may be used to identify sperm cells and distinguish sperm cells from debris and other objects in the sample. Individual sperm cells within an image may be tagged or coded for identification purposes. Additionally or alternatively, images may be compared longitudinally, i.e., multiple sequential images within the sequence may be analyzed, to track the location of individual sperm cells in multiple images. Register marks or other features in the images may be used to align multiple images in a sequence.

In a sequence of steps, sperm cells may be counted in one or more images to determine the sperm count for the semen sample. In the first step, individual sperm cells may be counted in one or more images in a sequence of images. As the images are taken from the detection zone of the microfluidic element of a cartridge, and the detection zone has a known volume, the sperm number for the semen sample, i.e., the number of sperm cells per unit volume, can be determined. Statistical reliability increases as sampling increases, so it may be advantageous to analyze multiple images in counting step. However, increased sample also puts a greater demand on the processor and can slow the overall analysis of the semen sample, so the number of images analyzed in step can be adjusted to reach the desired balance between reliability and speed of determining step. Thus, sperm cells may be counted in only a subset of captured images from video clips. For example, sperm cells may be counted only in 1 of every 2, 3, 4, 5, 6, 8, 10, or more images in a series.

In another sequence of steps, the information regarding the position of individual sperms in sequential images is used to determine sperm motility for the semen sample. First, the location of individual sperm cells is compared in sequential images. At least two sequential images should be compared, but any greater number of images can be analyzed as long as individual sperm cells can be tracked throughout the series. The positions are used to determine the rate and direction of movement of individual sperm cells. Because both rate and direction of movement are determined, movement of individual sperm cells may be represented by a vector. These values are compiled to determine sperm motility for the semen sample. The number of individual sperm cells tracked and the number of images in a series analyzed in steps and influence both the reliability and speed of determining step, so these values can be adjusted to reach the desired balance between reliability and speed in determining step

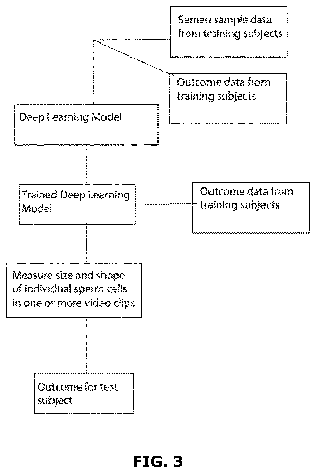

Fig. 3 depicts a deep learning component according to embodiments of the present invention. In simple term, the deep learning component may be configured to the deep learning component use data from semen samples to make diagnoses or prognoses regarding the fertility status of a subject or a course of treatment to treat infertility in a subject. The data from the semen samples may be raw image data, or it may be data produced from analysis of raw image data. For example, the semen sample data may comprise calculated values for sperm count, sperm morphology, sperm motility and sperm DNA integrity. The deep learning component may be configured to enter data from semen samples from subjects and outcomes, such as whether the subjects may be able to conceive and by what means, from those subjects into a deep learning component.

The deep learning system component may also be configured to train itself to represent features defined by the images and create a map of the outcome probabilities over the features. In some embodiments, the deep learning component may be deep learning component provided with the semen sample data and an associated outcome and allowed to autonomously identify features in the semen sample data in an unsupervised manner and then to identify predictive relationships between those identified features and the known outcome associated with the provided semen sample data. As data from subjects with known outcomes may be used to train the deep learning component, such data and subjects may be referred to as "training data" or "training subjects," respectively.

The deep learning component may also comprise a plurality of methods. For instance, the methods may comprise entering data from semen samples from subjects for whom outcomes are unknown into the deep learning component. As the outcomes of such individuals are unknown, such data and subjects may be referred to as "test data" and "test subjects," respectively.

In one embodiment, the deep learning component may identify predictive features in the test data, locate the features on the map of outcome probabilities, and/or provide a diagnosis and/or prognoses for the test subjects.

In one embodiment, the processor unit comprised by the analytical device may also be configured to receive optical data from the optical device. The processor unit may have sufficient power to perform a high-level, single-thread object detection and/or tracking.

Moreover, the processor unit may be single-core unit. In another embodiment, the processor unit may be a multi-core unit. Furthermore, the processor may comprise a speed of less than about 5 GHz, less than 3 GHz, less than 3 GHz, less than 500 MHz, such as, for example, or less than 250 MHz.

Fig. 3 further depicts steps of a method of evaluating male fertility using a deep learning component to analyze semen sample data according to embodiments of the present invention.

In simple terms, training data comprising semen sample data and/or outcome data, such as known fertility statuses, of the training subjects, may be provided to the deep learning component. Features may be detected within the training semen sample data by means of the deep learning component, which may (semi) autonomously learn associations between the features of and outcomes, thereby yielding a trained deep learning component. Test data, which may comprise semen sample data from a test subject, may then be provided to the trained deep learning component. The trained deep learning component may detect features such as DNA quality level, abnormalities in head morphology and abnormalities in tail movement pattern, in the test semen sample data, may correlate them with an outcome, e.g., a fertility status, and may provide an outcome for the test subject, such as the likelihood that the test subject will be able to conceive.

Moreover, the outcome data of the training subjects may be anything related to reproductive status. The outcome data may comprise treatment histories as well as results obtained from various types of treatment. Thus, for example, but not limited to, the outcome data may comprise information related to at least one of: medications, consumption of alcohol, tobacco, recreational drugs diet and dietary supplements including vitamins, physical activity levels including exercise and fitness regimens and periods of sitting, frequency of ejaculation, frequency and timing of intercourse, including the temporal relationship between intercourse and other factors, clothing and undergarments, use of hot tubs, baths, saunas and other environments that may expose testicles to non-physiological temperatures, surgeries including vasectomies and vasectomy reversals, hernia repairs, correction of undescended testicles, treatment of varicoceles, prostate surgery, and other testicular or abdominal procedures, mental and psychological factors including stress levels, exposure to environmental hazards such as industrial chemicals e.g. benzene, toluene, xylene, pesticides, herbicides, other organic solvents, painting materials, and the like, heavy metals or radiation, the number of fertilized eggs produced using subject's sperm, the number of eggs to which the subject's sperm was exposed, the method of introducing the sperm to eggs, e.g., intrauterine delivery, preparation of the sperm sample, in vitro fertilization (IVF), intracytoplasmic sperm injection (ICSI), etc., the number of fertilized eggs implanted, the number of fetuses observed at various gestational time points, the number of live births, the health status of the fetus or the newborn, and the like.

The system may comprise at least one computer. Optionally, the system may comprise a semen-analysis system, such as one described above, e.g., systems, and a server computer. Each computer in the system may comprise a processor unit coupled to a tangible, non-transitory memory device and at least one input/output device.

In one embodiment, the system may comprise at least one processor coupled to a memory subsystem such as a memory device or collection of memory devices. Moreover, the components, e.g., computer, server, and imaging system, may be in bidirectional communication over a network that may be wired or wireless and wherein the components may be remote or local.

In another embodiment, the system may be configured to receive and/or obtain training data, such as semen sample data and/or outcome data, as well as images of semen sample data generated by the semen analysis system and/or otherwise obtained. In a further embodiment, the system may use the memory to store the received data as well as the deep learning component data, which may be trained and otherwise operated by the processor unit.

The memory subsystem may comprise at least one or any combination of memory devices. The term memory device is intended to refer to a mechanical device configured to store data and/or instructions in a machine-readable format. Memory may comprise one or more sets of instructions, e.g., software, which, when executed by one or more of the processors of the disclosed computers can accomplish some or all of the methods or functions described herein. Preferably, each computer may comprise a no transitory memory device such as a solid-state drive, flash drive, disk drive, hard drive, subscriber identity module (SIM) card, secure digital card (SD card), micro SD card, or solid-state drive (SSD), optical and magnetic media, others, or a combination thereof.

Using the described components, the system is operable to produce a report and provide the report to a user via an input/output device. An input/output device is a mechanism or system for transferring data into or out of a computer. Exemplary input/output devices include a video display unit ( e.g., a liquid crystal display (LCD) or a cathode ray tube (CRT)), a printer, an alphanumeric input device ( e.g., a keyboard), a cursor control device ( e.g., a mouse), a disk drive unit, a speaker, a touchscreen, an accelerometer, a microphone, a cellular radio frequency antenna, and a network interface device, which can be, for example, a network interface card (NIC), Wi-Fi card, or cellular modem.

The deep learning component may be configured to receive images and video clips of semen samples and known outcomes, to identify features within the images in an unsupervised manner, and to create a map of outcome probabilities over the features.

In one embodiment, the deep learning component may be configured to receive images and video clips of semen samples from a test subject, identify within the test images predictive features learned from the training steps, and locate the predictive features on the map of outcome probabilities to provide a prognosis or diagnosis.

It should be understood that the term Deep learning is intended to refer to a class of machine learning operations that use a cascade of many layers of nonlinear processing units for feature extraction and transformation. Each successive layer may use the output from the previous layer as input. The algorithms may be supervised or unsupervised and applications include pattern analysis (unsupervised) and classification (supervised). Certain embodiments are based on unsupervised learning of multiple levels of features or representations of the data. Higher level features may be derived from lower level features to form a hierarchical representation. Such features may be preferably represented within nodes as feature vectors. Furthermore, the deep learning by a neural network may comprise learning multiple levels of representations that correspond to different levels of abstraction, levels form a hierarchy of concepts. In another embodiments, the neural network may comprise at least 5 and preferably more than 10 hidden layers. The many layers between the input and the output may advantageous, as it may allow the system to operate via multiple processing layers.

It should also be understood that the term deep learning may refer to part of a broader family of machine learning methods based on learning representations of data. An observation, e.g., an image, can be represented in many ways such as a vector of intensity values per pixel, or in a more abstract way as a set of edges, regions of particular shape, etc. Such features may be represented at nodes in the network. Preferably, each feature may be structured as a feature vector, a multi-dimensional vector of numerical features that represent some object. Moreover, such features may provide a numerical representation of objects, since such representations may facilitate processing and statistical analysis of data. Feature vectors may be similar to the vectors of explanatory variables used in statistical procedures such as linear regression. Feature vectors may often be combined with weights using a dot product in order to construct a linear predictor function that is used to determine a score for making a prediction. The vector space associated with those vectors may be referred to as the feature space. In order to reduce the dimensionality of the feature space, dimensionality reduction may be employed. Higher-level features can be obtained from already available features and added to the feature vector, in a process referred to as feature construction. Feature construction is the application of a set of constructive operators to a set of existing features resulting in construction of new features.

Within the network, nodes are connected in layers, and signals travel from the input layer to the output layer. In one embodiment, each node in the input layer may correspond to a respective one of the patches from the training data. The nodes of the hidden layer may be calculated as a function of a bias term and a weighted sum of the nodes of the input layer, where a respective weight may be assigned to each connection between a node of the input layer and a node in the hidden layer. The bias term and the weights between the input layer and the hidden layer may autonomously be learned in the training of the neural network. The network may comprise thousands or millions of nodes and connections. Typically, the signals and state of artificial neurons may comprise real numbers, typically between 0 and 1. Optionally, there may be a threshold function or limiting function on each connection and on the unit itself, such that the signal must surpass the limit before propagating. Back propagation is the use of forward stimulation to modify connection weights, and is sometimes done to train the network using known correct outputs.

The associations between features and outcome (e.g., fertility status) may be represented as a map of outcome probabilities where each identified feature is associated with a likelihood of the known outcome. The correlating step may comprise locating features detected in the test semen sample data on the predictive map of outcomes. The correlated pathology status may provide a prognosis or diagnosis for the test subject from which the test sample was obtained.

In another embodiment, the map of outcome probabilities may relate to the likelihood of a given outcome for an individual to one or more data points in a semen sample from the individual. The map may be established based on features identified in the training data as associated with various outcomes. Preferably, the map of outcome probabilities may be created autonomously by the deep learning system. Additionally or alternatively, the map may be created or refined with user input.

In case of providing diagnoses and/or prognoses a report may also be generated, which may be written and/or printed reports and/or may be electronic documents and may be transmitted, for example, to test subjects or to treating physicians either physically or electronically. Diagnoses may indicate an individual's fertility status. The diagnosis may be expressed as a probability or level confidence. The diagnosis may indicate a medical stage of a condition or disease. The diagnosis may comprise identification of an element in the semen sample data that is associated with low fertility or is amenable to a particular course of treatment. The diagnosis may comprise a recommended treatment regimen, such those described above.

While in the above, a preferred embodiment has been described with reference to the accompanying drawings, the skilled person will understand that this embodiment was provided for illustrative purpose only and should by no means be construed to limit the scope of the present invention, which is defined by the claims. Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

## Claims

1. A device for analyzing a seminal sample, the device comprising a microvolume unit configured to allocate at least one fluid, wherein the microvolume unit comprises
at least one inlet to receive the at least one fluid,
at least one outlet configured to expel the at least one fluid, and
at least one channel arranged on the microvolume unit,
wherein the at least one channel is configured to guide the at least one fluid through the microvolume unit.

2. The device according to the preceding claim, wherein the device comprises an inlet reservoir configured to hold the seminal sample, wherein the seminal sample comprises a raw-sperm sample, wherein the device comprises an outlet reservoir for collecting the sperm sample, wherein the sperm sample is separated from the raw sperm sample.

3. The device according to any of the preceding device claims, wherein the at least one channel comprises at least one selection channel arranged between the inlet reservoir and the outlet reservoir configured to provide a fluid connection between the inlet reservoir and the outlet reservoir, and wherein the at least one selection channel comprises
a channel height lower than 10µm, preferably lower than 5µm, more preferably lower than 3µm, such as 2µm,
a channel width between 10µm and 100um, preferably between 25µm and 150µm, more preferably between 50µm and 100µm.µm, and
a channel length between 1 and 10 cm, preferably between 3 and 8 cm, such as between 5 and 7 cm,
wherein the at least one selection channel is configured to
allocate a volume of at least 0.01 mL, preferably at least 0.02 mL, more preferably at least 0.05 mL and less than 1 mL, preferably less than 0.8 mL, more preferably less than 0.5 mL, and
allocate a volume of raw sperm sample comprising a concentration of least 10 million sperm per mL, preferably at least 50 million sperm per mL, more preferably at least 100 million sperm per mL, and less than least 250 million sperm per mL, preferably less than 100 million sperm per mL, more preferably less than 150 million sperm per mL.

4. The device according to any of the preceding device claims, wherein the device comprises
a detection zone configured to
allow the analysis of the separated seminal sample by means of the optical analytical device,
allocate a known volume of the seminal sample to count a number of sperm cells in the portion of the detection zone, and
at least one separation wall is arranged between the detection zone and the analytical device,
wherein the at least one separation wall comprises a transparent material configured to allow the at least light source passing through a portion of the separated sperm sample in the detection zone into an optical device.

5. The device according to any of the preceding device claims, wherein the device is further configured to simulate natural conditions of a female body via at least one of
a high-viscosity, and
physiological buffer in a micro-network,
wherein the device comprises at least one valve configured to assume at least two operational states, wherein the at least one valve is configured to regulate the fluidic connection between the device and the macrofluidic chamber, wherein the at least one valve is configured to assume a first operational state and a second operational state, wherein the first operational state and different from the second operational state, wherein the at least one valve is configured to
establish the fluid connection between the device and the microfluidic chamber while in the first operational state,
discontinue the fluid connection between the device and the microfluid chamber while in the second operational state, wherein the at least one valve component comprises at least one internal valve configured to regulate a flow within the device, wherein at least one of the at least one valve is a check valve configured to allow flow of fluids in only one direction through a conduit comprised by the check valve, wherein the at least one valve is configured to individually and/or independently be selected to assume at least one of the at least two operational state.

6. A method for analyzing a seminal sample using a device, the method comprising introducing the seminal sample into the device,
guiding the seminal sample through the device,
segregating sperm cells from the seminal sample into a fluid medium, and recovering sperm cells from the fluidic medium.

7. The method according to any of the 2 preceding claims, wherein introducing the seminal sample into the device further comprises introducing at least one fluid into the device through at least one inlet, wherein introducing the at least one fluid into the device further comprises expelling the at least one fluid through at least one outlet and wherein guiding the seminal sample through the device comprising the seminal sample through at least one selection channel comprised by the device.

8. The method according to any of the preceding method claims, wherein the method comprises supplying a flow of the at least one fluid in a medium flow direction, wherein the medium flow direction is also defined by an initial flow point and a terminal flow point, wherein the method comprises introducing the seminal sample into the device at the terminal flow point of the medium flow direction.

9. The method according to any of the preceding method claims, wherein the method comprises
recovering sperm cell from the fluidic medium at the initial flow point of the medium flow direction,
establishing a sorting threshold comprising a sperm profile, wherein the method comprises sorting the sperm cells based on the sorting threshold, and
analyzing sperm cells in the seminal sample using an analyzing component.

10. The method according to any of the preceding method claim, wherein the device comprises an inlet reservoir and outlet reservoir, wherein the method comprises
holding the seminal sample in the inlet reservoir, wherein the seminal sample comprises a raw-sperm sample,
collecting the sperm sample in the outlet reservoir,
separating the sperm sample from the raw sperm sample,
using a detection zone comprised by the device, and
allowing analysis of the separated seminal sample by means of an analytical device.

11. A system for analyzing seminal image data, the system comprising
a detecting component configured to detect sperm cells in the seminal sample,
an analyzing component configured to analyze sperm cells in the seminal sample,
a sorting component configured to sort sperm cells in the seminal sample, and
at least one deep learning component.

12. The system according to the preceding claim, wherein the at least one deep learning component is configured to use seminal image data to generate at least one finding comprising at least one of
a diagnose finding, and
a prognose finding,
information regarding the fertility status of at least one of
a subject, and
a course of treatment of the subject comprising at least one treatment approach to treat infertility in the subject.

13. The system according to any of the preceding system claims, wherein
the seminal image data comprises at least one of
raw image data from the seminal samples,
processed imaged data obtained by processing the raw image data, and the seminal sample data comprises at least one calculated value of at least one of
sperm count,
sperm morphology,
sperm motility, and
sperm DNA integrity.

14. An analytical method for evaluating male fertility using a system according to any of the preceding system claims, the method comprising
analyzing seminal sample,
training data comprising at least one of
seminal sample data,
finding data, wherein the finding data comprises at least one known fertility statuses,
detecting at least one feature within the training seminal sample data by means of the at least one deep learning component, and
using a test data comprising seminal sample data from a test,
wherein the analytical method comprises detecting the at least one feature by means of the at least one deep learning component, wherein the at least one feature is detected in test seminal sample data.

15. The analytical method according to the preceding claim, wherein the analytical method comprises correlating the at least one feature with at least one analytical finding, wherein the at least one analytical finding comprises at least one of
fertility status
treatment histories,
results obtained from various types of treatment,
related to at least one of
Age,
medications,
consumption of alcohol,
consumption of tobacco,
consumption of recreational drugs diet, and
consumption of dietary supplements including vitamins
physical activity levels including exercise and fitness regimens and periods of sitting,
frequency of ejaculation,
frequency and timing of intercourse including the temporal relationship between intercourse and other factors,
clothing and undergarments,
use of hot tubs, baths, saunas and other environments exposing testicles to non-physiological temperatures,
surgeries including vasectomies and vasectomy reversals, hernia repairs, correction of undescended testicles, treatment of varicoceles, prostate surgery, and other testicular or abdominal procedures,
mental and psychological factors including stress levels,
exposure to environmental hazards such as industrial chemicals e.g.
benzene, toluene, xylene, pesticides, herbicides, other organic solvents, painting materials, and the like, heavy metals or radiation,
number of fertilized eggs produced using subject's sperm,
number of eggs to which the subject's sperm was exposed,
method of introducing the sperm to intrauterine delivery, preparation of the sperm sample, in vitro fertilization (IVF), intracytoplasmic sperm injection (ICSI),
number of fertilized eggs implanted,
number of fetuses observed at various gestational time points, number of live births, and
health status of the fetus or the newborn,
wherein the analytical method comprises
providing for a test subject from which the seminal sample was obtained at least one of
a prognosis, and
a diagnosis,
generating a report comprising at least one of
the prognosis, and
the diagnosis,
selectively transmitting the report to at least one of
test subjects, and
treating physicians.
